Europäisches Patentamt

European Patent Office

Office européen des brevets

(19)

(11) Publication number: **0 269 382**

A1

## (12) EUROPEAN PATENT APPLICATION

(21) Application number: 87310266.9

(22) Date of filing: 20.11.87

(51) Int. Cl.⁴: **C12N 15/00** , C12N 5/00 , C07K 13/00 , A61K 37/02 , C12P 21/02

(30) Priority: 21.11.86 US 914215

(43) Date of publication of application: 01.06.88 Bulletin 88/22

(84) Designated Contracting States:
ES GR

(71) Applicant: SMITHKLINE BECKMAN CORPORATION
One Franklin Plaza P O Box 7929
Philadelphia Pennsylvania 19103(US)

(72) Inventor: Reff, Mitchell Elliot
423 Lori Lane
King of Prussia Pennsylvania 19406(US)

(74) Representative: Giddings, Peter John, Dr. et al
Smith Kline & French Laboratories Ltd.
Corporate Patents Mundells
Welwyn Garden City Hertfordshire AL7
1EY(GB)

(54) Recombinant protein expression.

(57) A mammalian cell line useful for production of a recombinant DNA product.

EP 0 269 382 A1

# RECOMBINANT PROTEIN EXPRESSION

## FIELD OF THE INVENTION

This invention relates to expression of gene products by recombinant DNA techniques in a mammalian host.

## BACKGROUND OF THE INVENTION

Human tissue Plasminogen Activator (tPA) is a glycoprotein of about 70,000 daltons and 527 amino acids. It is secreted by a variety of human tissues and is naturally present in serum. tPA functions in clot lysis by converting plasminogen to plasmin. tPA has an affinity for and is more active in a presence of fibrin. Thus, tPA is useful in treating disorders associated with thrombi formation such as deep vein thrombosis, acute myocardial infarction and pulmonary embolism. See, generally, Rijken et al., Biochem. Biophys. Acta 580:140 (1979), Vetterlein et al., J. Biol. Chem. 254:575 (1979), Rijken et al., J. Biol. Chem. 256:7035 (1981) and Matsuo et al., Nature 291:590 (1981).

tPA is overproduced by certain cell lines, including the Bowes melanoma cell line. See, e.g., Wilson et al., Canc. Res. 40:933 (198). Collen et al., European Patent Application (EP-A)-41,766, disclose purification of Bowes melanoma-derived tPA. Wilson, EP-A-113,319, disclose production and purification of tPA using a serum-independent Bowes melanoma cell line. Brouty-Boye, Bio/Technology, December 1984, p. 1058, report production of tPA by human embryonic lung cells. Schleuning et al., Sixth Int'l. Conf. Fibrinolysis, Lausanne, Switzerland, Abstract, July 22-23, 1982 report production of tPA by HeLa cells.

tPA can also be prepared by recombinant DNA techniques. Isolation of mRNA for tPA is disclosed, e.g., by Opdenakker et al., Eur. J. Biochem. 121:269 (1982). Isolation of cDNA for part of tPA is disclosed by Edlund et al., Proc. Natl. Acad. Sci. USA 80:349 (1983). Cloning of cDNA for tPA in E. coli is reported by Pennica et al., Nature 301:214 (1983). Cloning of cDNA for tPA in E. coli and in Chinese Hamster Ovary cells by application of routine recombinant DNA procedures is disclosed by Goeddel et al., EP-A-93,619 and by Levinson et al., EP-A-117,059. Goldberg et al., PCT patent application W085-03949, disclose expression of tPA in E. coli. Meyhack et al., EP-A-143,081, disclose expression of tPA in yeast. Robinson, W084-01786, discloses a modified tPA lacking all or a portion of the carbohydrate moieties present in native tPA.

Howley et al., U.S. Patent 4,419,446, disclose use of BPV DNA, including the 69T region thereof, as a cloning vector in mammalian cells. Ricciardi et al., U.S. Patent 4,562,155, disclose plasmid pML BK, a plasmid having E. coli and mammalian (BK virus) maintenance functions.

The HAK, or HaK, is a hamster kidney cell line which was originally isolated in 1959 by I.M. Spense from kidneys of two normal young adult male Syrian hamsters. The origin and characteristics of the HAK cell line are described in the Catalogue of Bacteria, Phages and rDNA Vectors, 16th edition, 1985, American Type Culture Collection, Rickville, Maryland, under accession number CCL-15. The HAK cell line has not previously been reported as a host for transfection with foreign DNA.

## SUMMARY OF THE INVENTION

The invention is a HAK cell comprising a tPA gene expression unit.

The invention is also a process for preparing a cell culture for production of tPA which comprises transfecting a HAK cell with a tPA gene expression unit and selecting and propagating transfectants which produce tPA.

The invention is also tPA produced by a recombinant HAK cell and a pharmaceutical composition comprising a thrombolytic, non-toxic amount of such tPA.

The invention is also a recombinant DNA cloning vector which can be used in the process for preparing the HAK cell of the invention and a HAK cell transfected with such vector.

## BRIEF DESCRIPTION OF THE DRAWINGS

Fig. 1 is an illustration of BPVneo, a BPV-pML2 hybrid vector having a neomycin resistance cassette.

Fig. 2 is an illustration of Mbaneo.ltPA vector having a tPA gene expression unit cassette and a neomycin resistance cassette.

## DETAILED DESCRIPTION OF THE INVENTION

It has now been found that upon introduction of a tPA gene expression unit into a HAK cell by transfection techniques, such transfected HAK cell produces an unexpectedly large amount of tPA relative to copy number of the tPA gene expression unit. For example, HAK clone 2.8, described herein below, was shown to produce about 3-5 pg of tPA per single tPA gene copy, which amount is about 20-fold greater than the amount of tPA per tPA gene copy produced by Chinese hamster ovary (CHO) cells.

The tPA gene expression unit which can be used to prepare the recombinant HAK cell of the invention comprises a DNA coding sequence for tPA, or for an active variant thereof, and a regulatory region necessary or desirable for transcription of the tPA coding sequence and for subsequent translation. The tPA coding sequence, or the variant thereof, is available from several sources or can be obtained from human cells or cell lines, such as the Bowes melanoma cell line or the HeLa cell line. Standard techniques of genetic engineering are utilized to derive a tPA coding sequence from the genomic DNA of a human cell. Variants thereof can also be prepared by standard recombinant DNA and/or synthetic techniques. References cited in the background section, above, are illustrative.

The regulatory region can be one which is commonly employed in construction of mammalian cell expression vectors, or a region which performs similar functions. Such region typically comprises a promoter region, that is, a region which functions in binding of RNA polymerase and RNA transcription initiation, upstream of the tPA coding sequence. Promoters which are commonly employed in mammalian cell expression vectors and which, therefore, are useful in the present invention include the metallothionein promoter, especially the monkey, mouse and human metallothionein promoter; viral promoters such as the Simian Virus 40 (SV40) early promoter or the SV40 late promoter; viral LTR's, such as the LTR of mouse mammary tumor virus (MMTV), the LTR of human T-cell lymphotrophic viruses (HTLV) I, II or III or the LTR of rous sarcoma virus. Such promoters can be derived from genes or cells in which they are naturally present. Such promoters can also be modified to alter their sequence.

Downstream of the tPA coding sequence, there is typically a polyadenylation (poly A) region, that is, a region which functions in polyadenylation of RNA transcripts. Such poly A region apparently plays a role in stabilizing transcription. Polyadenylation regions which are commonly employed in mammalian cell expression vectors and which, therefore, are useful in the present invention include the polyadenylation regions of the SV40 early region, bovine growth hormone and human collagen pro-alpha 2(I). Such regions can be derived from the genes in which they are naturally present. Such regions can also be modified to alter their sequence provided that polyadenylation and transcript stabilization functions are not significantly adversely affected.

Other regulatory functions, including splicing sites, transcriptional activators and enhancers, can also be comprised within the gene expression unit or elsewhere within a DNA molecule comprising the gene expression unit.

A HAK cell is transfected with the tPA gene expression unit by any technique that permits introduction of foreign DNA into a host cell without significantly adversely affecting the foreign DNA or the host cell. Such techniques include calcium precipitation, electroporation, protoplast fusion, microinjection and viral transfection.

The tPA gene expression unit preferably is incorporated into a larger DNA molecule prior to transfection. Such larger DNA molecule preferably comprises at least a genetic selection marker in addition to the tPA functions. The selection marker can be any gene or genes which cause a readily detectable phenotypic change in a transfected host cell. Such phenotypic change can be, for example, foci formation as can occur when a bovine papilloma virus (BPV) vector is employed; drug resistance, such as genes for G418 or hygromycin B resistance; or other selectable markers such as xanthine guanine phosphoribosyl transferase (xgprt), thymidine kinase (TK) and galactokinase (galK). A selection marker which supplies amplification functions can be employed to increase copy number, whether by increased transfection efficiency or by enhanced intra-cellular replication, or the marker and of the tPA gene expression unit. Such markers which also serve to amplify gene copy number include genes for dihydrofolate reductase (methotrexate resistance), CAD (N-phosphonacetyl-L-aspartate resistance) and adenosine deaminase (2-deoxycorfomycin re-

3

sistance).

The larger DNA molecule comprising the tPA gene expression unit can be a plasmid which may or may not be capable of episomal maintenance in a HAK transfectant. Such plasmid which is capable of stable, episomal maintenance will comprise, in addition to the tPA gene expression unit, a maintenance function, that is, a region which causes the DNA to replicate independently of mitotic chromosomal replication, in a HAK cell. Such maintenance functions are typically of viral origin, including, for example, animal viruses such as bovine papilloma virus (BPV), Simian Virus 40 (SV40), BK virus (BKV), adenovirus, Epstein Barr Virus (EBV), polyoma viruses and herpes simplex virus (HSV). Such maintenance functions can be readily prepared and tested for stable, episomal maintenance in a HAK cell. Plasmid, and therefore tPA gene, copy number is typically in the range of 1 - 1000, most often 1 - 200.

Plasmids within which a tPA gene expression unit can be incorporated are available from many sources or can be constructed by techniques well-known in the art of gene expression in mammalian hosts by recombinant DNA techniques. Among the sources are public depositories, including the American Type Culture Collection, Rockville, Maryland, which includes several useful plasmids in its catalog.

The tPA gene expression unit can be co-transfected with other DNA functions, such as a gene for an amplifiable function as described above. Such other DNA function can be linked directly or indirectly to the gene expression unit or it can be unlinked, that is, on a different molecule. See, for example, Axel, U.S. Patent 4,399,216.

Following transfection, a cell which carries the tPA gene expression unit is cultured in a nutrient medium under conditions such that recoverable quantities of tPA are produced. Standard mammalian cell culture medium can be employed, for example, Morgan, Morton and Parker's medium 199 plus 10% bovine serum; Earle's BSS (87%), 2.5% lactalbumin (0.5%) and 10% horse serum; Eagle's basal medium and 5% calf serum; and, Eagle's basal medium with Hanks' BSS and 10% calf serum. HAK cells grow well in simple serum-free medium. Cell cultures are maintained at ambient pressure at 30 to 45°C, preferably at 35 to 42°C and most preferably at 37°C.

A standard protocol includes transformation with plasmid DNA comprising a tPA gene expression unit by the calcium precipitation technique followed, after about 6-8 hours incubation, by a glycerol shock treatment. Transformation typically results in the host cell having up to about 20 copies of the tPA gene expression unit. Amplification techniques can be used to increase that copy number up to about 1000 and typically up to about 200. About 24 hours after transformation, the cells are placed in selective medium and cultured for about 2 weeks. Surviving colonies are placed in 24 well micro-titer plates and incubated until confluency, i.e., about 1/2 to 1 week. The wells are then assayed for tPA production. High-producing colonies are then expanded by successive incubations, each about 1 week, in T25 flasks, T75 flasks, roller bottles and, finally, production units. In production units, conditioned medium can be circulated or periodically withdrawn and replaced so that tPA can be harvested continuously or periodically. Production units are preferably any culture system designed for adherent cells, cell culture units which employ fibers, microcarriers or microcapsules and cell factories.

Following cell culture, tPA is isolated from conditioned medium by standard protein isolation techniques. These typically involve a series of chromatography procedures including, for example, ion exchange, reverse phase and affinity chromatography procedures. Following purification, the tPA is formulated into a pharmaceutical composition such that each dosage unit comprises an amount of tPA which is non-toxic, that is, free of significant adverse side effects, and which is effective in causing thrombolysis following administration to an animal, particularly man. Such pharmaceutical composition for treatment of myocardial infarction typically is in a form suitable for intravenous injection or infusion and comprises 0.1 to 10 mg/ml of tPA in a pharmaceutically acceptable diluent or carrier. A typical treatment for an adult person suffering from acute myocardial infarction comprises intravenous infusion of 80-150 mg of tPA over a period of .25-6 hours. Suitable diluents and carriers are well-known to a pharmaceutical chemist, as are techniques for preparing the pharmaceutical composition.

EXAMPLES

The examples which follow are illustrative, and not limiting, of the invention.

## EXAMPLE 1. BPVβglobinneotPAB

A cDNA coding sequence which had been prepared by reverse transcription of mRNA from HeLa cells {Schleuning et al., Sixth Int'l. Conf. Fibrinolysis, Lausanne, Switzerland, July 22-23, 1982) was inserted in-frame between the SV40 early protein promoter and a BGH polyadenylation site (Woychik et al., Biochem. 81:3944 (1984); Goodwin et al., EP-A-173,552) to prepare a tPA gene expression unit. The tPA gene expression unit was bounded on both ends by a SalI restriction endonuclease cleavage site.

The SalI cassette, which comprised the tPA gene expression unit, was inserted into a vector, BPVβglobinneo. The vector, BPVβglobinneo, consists of (i) BPV-1 DNA linearized at the BamHI site, (ii) pBR322 replication functions and the β-lactamase gene both of which had been derived from pML2 (Melon et al., Cell 27:279-288 (1981)) by deleting a non-essential BamHI-SalI region, and (iii) a neomycin resistance selection marker (neo) which had been prepared by fusion of the β-globin promoter, a neomycin phosphotransferase coding sequence and the SV40 early region polyadenylation region. The tPA gene expression unit was inserted into a SalI site between the BPV DNA and the pML2 DNA.

Competent E. coli HB101 cells were transformed with the vector, referred to as BPVβglobinneotPA, "A" or "B" depending upon orientation of the tPA cassette, and ampicillin resistant clones were selected. Clones having the circular plasmid (A), which is illustrated as follows:

```
      ←────────              ─────────→                    ←──── A ────
          BPV                   neo            pML2               tPA

   ├──────────────┼──────────────────┼──────────────┼──────────────┼──────────────┤
        SalI              BamHI           BamHI            SalI           SalI
```

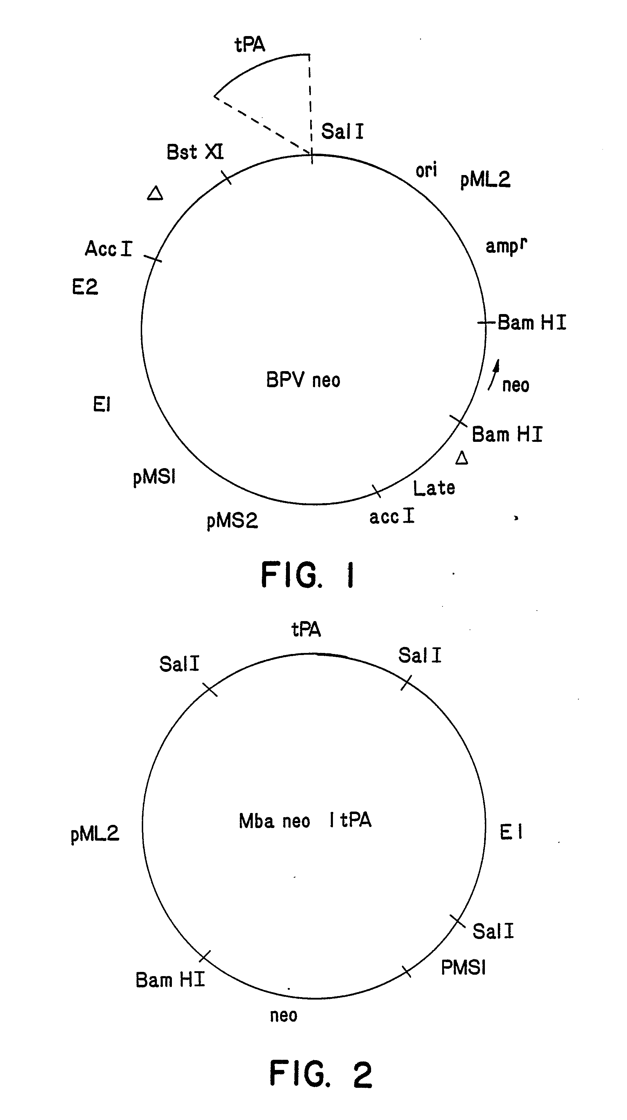

were cultured and plasmid DNA was isolated therefrom. (Arrows indicate direction of transcription.) See, Fig. 1.

HAK cells were transfected with BPVβglobinneo tPAB by calcium phosphate precipitation followed 6 hours later by glycerol ·shock. Substantially as described by Wigler et al., Proc. Natl. Acad. Sci. USA 76:1375-1376 (1979). Cells were incubated at 37°C overnight and then were treated with approximately 300 μm/ml of G418. Surviving colonies were picked and grown up in a 24 well microtiter dish for 3 days at 37°C. The supernatant from confluent cells was shown to contain active tPA which had been processed into the medium by the S-2251 assay. The amount of such exported tPA was about 0.5 pg/cell/day. The S-2251 assay is described by Weinberg et al., J. Immunol. Meth. 75: 289 (1984). Based on Southern Blot analysis, it appears that the plasmid is present in single copy. The plasmid was not integrated, as evidenced by absence of junction fragments following treatment with various restriction endonucleases and Southern Blot Analysis. The HAK cells transfected with the plasmid vector are referred to as HAK(BPVβglobinneotPAB).

This example thus demonstrates production of about 0.5 pg/day of tPA per single copy of tPA gene expression unit on a plasmid vector having maintenance functions derived from BPV.

## Example 2. Mbaneo.ItPA

The vector, BPVΔβglobinneo was cut with HindIII, filled in and then cut with ScaI to isolate a 432 base pair fragment containing plasmid maintenance sequence[1] (PMS1).

This fragment was inserted into a filled in BamHI site in βglobinneo behind the neo cassette. βglobinneo is a vector containing (i) a neomycin resistance selection marker (neo) which has been prepared by fusion of the β-globin promoter, a neomycin phosphotransferase coding sequence, and the SV40 early polyadenylation region, and (ii) pBR3222 replication functions and the β-lactamase gene. The resulting vector was βglobinneoPMS1.

This vector was cut with BamHI and SalI to isolate a fragment containing the neo function and PMS1. A second fragment (BamH1 to Sal1) consisting of pML2 DNA was isolated from BPVβglobinneo. These were ligated in a presence of a third fragment consisting of a 2608 base pair E1 protein gene expression unit having SalI ends. The E1 gene expression unit was previously prepared by ligating a NruI-NcoI fragment comprising the E1 coding sequence to the SV40 early promoter and the SV40 polyadenylation region. E. coli HB101 transformants were selected for plasmids having all three fragments. This plasmid was designated Mbaneo.I.

Into the SalI site in Mbaneo.I was inserted a tPA gene expression unit to prepare Mbaneo.ItPA. The

gene expression unit was contained on a 3046 base pair Sall fragment. It contained the SV40 early promoter and the BGH polyadenylation region. E . coli HB101 transformants having plasmid Mbaneo.ltPA (See Fig. 2) were selected.

HAK cells were transfected with Mbaneo.ltPA substantially as described above. From a first transfection, 6 out of 15 colonies that were G418 resistant produced tPA. One of these produced relatively high amounts of tPA. A sub-clone thereof, identified herein as HAK 2.8 was shown to produce 3-5 pg of tPA per cell per day. A sample of HAK 2.8 was deposited in the Americal Type Culture Collection, Rockville, Maryland, under accession number CRL 9182. This clone contains a single integrated copy of the tPA gene expression unit and, thus, produces 3-5 pg of tPA per tPA gene expression unit, per cell per day.

From a second transfection, 10 out of 10 colonies produced about 500 ng/ml of tPA. One produced about 3000 ng/ml. A sub-clone of the high producing clone, identified herein as HAK 1, was shown to produce about 1-2 pg of tPA per cell per day and to contain about 10-15 copies of the tPA gene expression unit, integrated.

From a third transfection, 39 out of 40 colonies expressed low levels of tPA. One, HAK 21, produced about 500 ng/ml. This is equivalent to about 0.5 pg of tPA per cell per day. HAK 21 also contains about 10-15 copies of the tPA gene expression unit, integrated.

The levels of expression per gene copy achieved in the case of HAK 2.8 are unexpectedly high in view of experience with other mammalian cells transfected with tPA gene expression units, such as CHO transfectants. Thus, this example demonstrates expression of tPA in HAK cells from a tPA gene expression unit integrated in low copy number but capable of high levels of tPA production relative to gene copy number. By amplification of the copy number of the tPA gene expression unit, increased levels of tPA expression are obtainable.

The above description and examples fully disclose the invention including preferred embodiments thereof. The invention, however, is not limited to precisely the constructions specifically disclosed but, rather, encompasses all modifications thereof coming with the scope of the claims which follow.

## Claims

1. A HAK cell comprising a tissue Plasminogen Activator (tPA) gene expression unit.

2. The HAK cell of claim 1 in which the tPA gene expression unit is episomal and is present in 1-1000 copies.

3. The HAK cell of claim 2 in which the episomal DNA is a plasmid having viral maintenance functions.

4. The HAK cell of claim 3 in which the plasmid maintenance functions are derived from an animal virus selected from the group consisting of bovine papilloma virus (BPV), Simian Virus 40 (SV40), herpes simplex virus (HSV), adenovirus, Epstein Barr Virus (EBV), polyoma virus and BK virus (BKV).

5. The HAK cell of claim 4 in which the plasmid comprises maintenance functions derived from BPV.

6. The HAK cell of claim 4 in which the promoter in the tPA gene expression unit is derived from a gene for metallothionein, the LTR of mouse mammary tumor virus (MMTV), the SV40 late or early promoter, the LTR of HTLV viruses or the LTR of rous sarcoma virus.

7. The HAK cell of claim 5 in which the tPA gene expression unit comprises the SV40 early promoter and the bovine growth hormone (BGH) or SV40 early polyadenylation region.

8. The HAK cell of claim 1 in which the tPA gene expression unit is integrated and is present in 1-1000 copies.

9. The HAK cell of claim 8 in which the promoter in the tPA gene expression unit is derived from a gene for metallothionein, the LTR of MMTV, the SV40 late or early promoter, the LTR of HTLV viruses or the LTR of rous sarcoma virus.

10. The HAK cell of claim 9 in which the tPA gene expression unit comprises the SV40 early promoter and the BGH or SV40 early polyadenylation region.

11. The HAK cell of claim 1 which is an HAK 2.8 cell.

12. A process for preparing a cell culture for production of tPA which comprises transfecting a HAK cell with a tPA gene expression unit and selecting and propagating transfectants which product tPA.

13. The process of claim 12 in which the HAK cell is transfected with a plasmid capable of episomal maintenance in the host cell in 1-1000 copies.

14. The process of claim 13 in which the plasmid comprises viral maintenance functions.

15. The process of claim 14 in which the promoter in the gene expression unit is derived from a gene for metallothionein, the LTR of MMTV, the SV40 early or late promoter of the LTR of HTLV viruses.

16. The process of claim 15 in which the plasmid maintenance functions are derived from BPV.

17. The process of claim 15 in which the tPA gene expression unit comprises the SV40 early promoter and the BGH or SV40 early polyadenylation region.

18. The process of claim 12 which comprises transfecting the HAK cell such that the tPA gene expression unit is integrated into the host cell genome.

19. The process of claim 18 which comprises amplifying the tPA gene expression copy number up to about 1000.

20. The process of claim 19 in which the promoter in the tPA gene expression unit is derived from a gene for metallothionein, the LTR of MMTV, the SV40 late or early promoter or the LTR of HTLV viruses.

21. The process of claim 20 in which the tPA gene expression unit comprises the SV40 early promoter and the BGH or SV40 early polyadenylation region.

22. The process of claim 12 in which the resulting HAK cell is a HAK 2.8 cell.

23. The process of claim 20 which comprises amplifying the tPA gene expression unit by co-transfection thereof with an amplifiable function.

24. The process of claim 23 in which the amplifiable function is a gene for dihydrofolate reductase, CAD or adenosine deaminase.

25. tPA produced by a recombinant HAK cell.

26. A pharmaceutical composition for effecting thrombolysis in a human subject which comprises an effective, non-toxic amount of the tPA of claim 25 and a pharmaceutical carrier therefor.

FIG. 1

FIG. 2

| | DOCUMENTS CONSIDERED TO BE RELEVANT | | EP 87310266.9 |

| Category | Citation of document with indication, where appropriate, of relevant passages | Relevant to claim | CLASSIFICATION OF THE APPLICATION (Int. Cl.4) |
|---|---|---|---|
| P,A | EP - A1 - 0 211 260 (KANEGAFUCHI KAGAKU KOGYO KABUSHIKI KAISHA)<br>* Claims 1-5,15,21,22,30 * | 1,6,7, 12,17 | C 12 N 15/00<br>C 12 N 5/00<br>C 07 K 13/00 |
| D,A | EP - A1 - 0 173 552 (THE UPJOHN COMPANY et al.)<br>* Claims 1,6,8,9 * | 1,6,7 | A 61 K 37/02<br>C 12 P 21/02 |
| D,A | EP - A2 - 0 143 081 (CIBA-GEIGY AG)<br>* Claims 1,3,39 * | 1,26 | |
| D,A | EP - A2 - 0 117 059 (GENENTECH, INC)<br>* Claims 1,2,8 * | 1,12 | |
| D,A | EP - A1 - 0 093 619 (GENENTECH, INC)<br>* Claims 1,3,9,13,14 * | 1,12, 19 | TECHNICAL FIELDS SEARCHED (Int. Cl.4) |
| A | CHEMICAL ABSTRACTS, vol. 104, no. 15, April 14, 1986, Columbus, Ohio, USA<br><br>M.L.S. LEDBETTER et al. "Cooperation between epithelial cells demonstrated by potassium transfer"<br>page 455, right column - page 456, left column, abstract-no. 127 160y<br><br>& Am.J.Physiol. 1986, 250 (2, Pt.1), C 306 - C 313 | 1 | C 12 N<br>C 07 K<br>A 61 K<br>C 12 P |

The present search report has been drawn up for all claims

| Place of search | Date of completion of the search | Examiner |
|---|---|---|
| VIENNA | 26-01-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82